# EUROPÄISCHE PATENTSCHRIFT

(11) **EP 0 413 265 B1**
(45) Veröffentlichungstag und Bekanntmachung des Hinweises auf die Patenterteilung: **13.04.1994**
(21) Anmeldenummer: 90115345.2
(22) Anmeldetag: 10.08.1990
(51) Int. Cl.: B01D 63/02, C08G 18/10, C08G 18/66, C08G 18/48, C08G 18/76

(54) **Transparente, heissdampfsterilisierbare, kompakte Polyurethan-Vergussmassen, Verfahren zu ihrer Herstellung und ihre Verwendung, insbesondere für medizinisch technische Artikel**
Transparent, steam-sterilisable, compact polyurethane casting materials, process for their preparation and their use, in particular for medical technical articles
Masses compactes coulées, transparentes de polyuréthane stérilisables par vapeur chaude, procédé pour leur préparation et leur utilisation, en particulier pour des articles techniques medicaux

(30) Priorität: 18.08.1989 DE 3927244
(43) Veröffentlichungstag der Anmeldung: 20.02.1991
(73) Patentinhaber: BASF Aktiengesellschaft, 67063 Ludwigshafen (DE)
(72) Erfinder: Horn, Peter, Dr., D-6900 Heidelberg (DE); Hinz, Werner, Dr., D-6710 Frankenthal (DE); Heckmann, Walter, Dr., D-6940 Weinheim (DE); Ramsteiner, Falko, Dr., D-6700 Ludwigshafen (DE); Gerold, Friedrich, D-8013 Haar (DE)

(56) Entgegenhaltungen:
- EP-A- 0 111 121
- DE-A- 2 749 491
- FR-A- 2 380 309
- US-A- 3 962 094
- US-A- 4 224 164
- US-A- 4 629 768
- US-A- 4 814 103

## Beschreibung

Erfindungsgegenstand sind transparente, heißdampfsterilisierbare, im wesentlichen kompakte Polyurethan- - im folgenden auch abgekürzt PU genannt - Vergußmassen, die hergestellt werden durch Umsetzung von
A) modifizierten Diphenylmethan-diisocyanaten - im folgenden auch abgekürzt MDI genannt -, die ihrerseits erhalten werden durch Umsetzung von
   A1) 4,4'-MDI oder
   A2) 2,4'-MDI oder
   A3) einer MDI-Mischung, die bezogen auf 100 Gew.-Teile, vorteilhafterweise besteht aus
      A31) 20 bis 90 Gew.-Teilen 4,4' -MDI,
      A32) 80 bis 8 Gew.-Teilen 2,4' -MDI und
      A33) 0 bis 5 Gew.-Teilen 2,2' -MDI,
mit
A4) mindestens einem Polyoxypropylen-polyol mit einer mittleren Funktionalität von 4 bis 8 und einer Hydroxylzahl von 230 bis 500, erhalten unter Verwendung von Sorbit, Sucrose oder Mischungen aus Sorbit und Sucrose als Startermoleküle, wobei als Costarter zusätzlich Wasser, Propylenglykol, Glycerin oder Mischungen aus mindestens 2 der Costarter mitverwendet werden können oder
A5 mindestens einem mit Sucrose oder vorzugsweise Sorbit gestarteten Polyoxypropylen-polyoxyethylen-polyol mit einem Gehalt an polymerisierten Ethylenoxideinheiten von 1 bis 80 Gew.-%, bezogen auf das Gesamtgewicht, und einer Hydroxylzahl von 150 bis 500 oder
A6) einer Polyoxyalkylen-polyolmischung mit einer mittleren Funktionalität von mindestens 4, hergestellt aus mindestens einem der vorgenannten Sucrose- oder Sorbit-polyoxypropylen (A4)- oder -polyoxypropylenpolyoxyethylen-polyole (A5) und einem Polyoxypropylen- und/oder Polyoxyethylen-polyol mit einer Hydroxylzahl von 350 bis 950, erhalten durch Umsetzung von Glycerin, Trimethylolpropan oder einer Mischung aus Glycerin und Trimethylolpropan mit 1,2-Propylenoxid oder Ethylenoxid im Molverhältnis 1:1 bis 1:8 oder Mischungen aus mindestens zwei der Komponenten (A4) bis (A6)
im Verhältnis von NCO-:OH-Gruppen von 2,5:1 bis 15:1,
mit
B) mindestens einer Verbindung mit mindestens zwei reaktiven Wasserstoffatomen, die vorzugsweise keine primären, sekundären oder tertiären Aminogruppen gebunden enthalten,
in Gegenwart oder Abwesenheit von
C) Katalysatoren.

PU-Gießsysteme sind bekannt und werden z.B. zusammenfassend beschrieben im Kunststoff-Handbuch "Polyurethane", Band 7, 2. Auflage, 1983, Seiten 392ff, herausgegeben von Dr. G. Oertel, im Carl Hanser Verlag, München, Wien.

Die Verwendung von PU-Vergußmassen zur Herstellung von Formteilen für medizinisch technische Geräte, insbesondere als Einbettwerkstoff für Hohlfasern in Dialysatoren, ist ebenfalls nicht neu und wird aufgrund der einfachen Handhabung von PU-Vergußmassen sowie deren geringer Schrumpfung während des Aushärtungsprozesses als vorteilhaft empfohlen. Bekannt sind z.B. folgende PU-Formulierungen speziell zur Einbettung von Hohlfasern.

In der US-A-3 962 094 werden katalysatorfreie Vergußmassen aus Rizinusöl-4,4' -MDI-, -Toluylen-diisocyanat- oder -Phenylen-diisocyanat-Prepolymeren mit endständigen NCO-Gruppen und einem Vernetzungsmittel, das enthält Rizinusöl und/oder einen Ester aus einem mindestens vierwertigen Alkohol und einer Hydroxy- oder Epoxygruppen aufweisenden aliphatischen Carbonsäure mit mindestens 12 Kohlenstoffatomen, beschrieben.

Nach Angaben der DE-A-2 749 491 (US-A-4 170 559) bestehen die katalysatorfreien Vergußmassen aus einem Prepolymeren, hergestellt aus Rizinusöl und Polyoxypropylen-glykol sowie 4,4' -MDI, und einem Vernetzungsmittel auf der Grundlage eines Esters aus einem mehrwertigen Alkohol mit 2 oder 3 Hydroxylgruppen und einer aliphatischen Carbonsäure mit mindestens 12 C-Atomen und einer oder mehreren Hydroxyl- und/oder Epoxygruppen. Als geeignete Polyisocyanate zur Herstellung der Prepolymeren werden ferner genannt: 2,4- und 2,6-Toluylen-diisocyanat oder Phenylen-diisocyanat. Als Vernetzungsmittel kommen außerdem Monoester und/oder Diester von Ethylenglykol und Ricinolsäure, Trimethylolpropan oder -ethan in Betracht.

Physiologisch unbedenkliche PU-Formstoffe, insbesondere zum Einbetten von Hohlfasern in Dialysatoren, werden nach Angaben der DD-A-251 565 hergestellt durch Umsetzung hochreaktiver, niedrigviskoser und lagerstabiler Mischprepolymerer, bestehend aus festen, hochreaktiven aromatischen Diisocyanaten und weniger reaktiven, flüssigen Diisocyanaten im Gewichtsverhältnis von 1:5 bis 5:1 und Polyolen, mit Polyolen aus der Gruppe Rizinusöl und/oder dessen Umesterungsprodukte, hochreiner Polyester und Polyoxytetramethylen-glykol. PU-Vergußmassen aus einem Isocyanatendgruppen aufweisenden PU-Prepolymeren und einer N,N,N' ,N' -Tetrakis(2-hydroxypropyl)-ethylendiamin enthaltenden Polyolmischung sind Gegenstand des US-Patents Hr. 4 224 164. Nach Angaben der US-A-4 742 112 finden zur Herstellung von PU-Vergußmassen für elektrische Geräte als Polyolkomponente Mischungen aus 10 bis 60 Gew.% eines Ricinolsäureesters und 90 bis 40 Gew.% eines C₂- bis C₆-Kohlenwasserstoffpolymeren mit mindestens einer Hydroxylgruppe Verwendung. Zweikomponenten PU-Formulierungen, die im gehärteten Zustand nicht cytotoxisch sind und sich als Vergußmassen für Trennvorrichtungen eignen, bestehen nach Angaben der DE-A-3 048 529 (US-A-4 332 927) aus mindestens einem NCO-terminiertem Prepolymeren, mindestens einem Polyol und einer katalytisch wirksamen Mengen einer dicarboxylierten Dialkylzinnverbindung. Mit Zinn-Schwefel-Verbindungen katalysierte PU-Vergußmassen zur Einbettung von Cellulosehohlfasern in Dialysatoren werden beschrieben in der DD-A-155 777.

Die vorgenannten PU-Vergußmassen können zu medizinisch-technischen Geräten oder Formteilen hierfür verarbeitet und vor ihrem Gebrauch mit Ethylenoxid oder/und mit γ-Strahlen sterilisiert werden. Nachteilig an dieser Art der Sterilisation ist jedoch, daß verbleibende Ethylenoxidspuren bei Patienten teilweise Allergien auslösen können und die γ-Strahlen nicht identifizierbare Spaltprodukte bilden können, so daß beim Patienten ein gewisses Gesundheitsrisiko, verursacht durch die Dialyse, nicht vollständig ausgeschlossen werden kann. Die nach dem Stand der Technik bekannten Vergußmassen sind ferner nicht ausreichend temperatur- und chemikalienresistent, so daß diese keiner Heißdampfsterilisation bei einer Temperatur von 121°C über einen Zeitraum von 20 Minuten unterworfen werden können.

Ein anderer gravierender Nachteil besteht darin, daß die bekannten PU-Vergußmassen nicht mit jedem Fasertyp verarbeitbar sind. So werden beispielsweise Cellulosefasern von PU-Vergußmassen auf Rizinusölbasis angegriffen und geschädigt. Außerdem treten bei der Verarbeitung der zum Stande der Technik gehörenden PU-Vergußmassensysteme vielfach Schwierigkeiten während des Produktionsablaufs auf. Die hergestellten Vergußmassen lassen sich zwar unmittelbar nach dem Verguß und innerhalb eines Zeitraums von ungefähr 30 Minuten danach schneiden; sie härten jedoch sehr rasch nach, so daß die Formkörper, vorzugsweise Dialysefilter bereits nach 24 Stunden nicht mehr geschnitten werden können. Dieses nachteilige Verhalten führt insbesondere bei Produktionsstörungen und an Arbeitsenden zu Produkteinbußen. Finden zur Herstellung der PU-Vergußmassen PU-Formulierungen Verwendung, die als Aufbaukomponenten primäre, sekundäre und/oder tertiäre Aminogruppen und reaktive Wasserstoffatome gebunden enthalten, so sind die erhaltenen Vergußmassen instabil gegen eine Sterilisation mit Peressigsäure. PU-Vergußmassen auf Rizinusölbasis werden hingegen an den Doppelbindungen oxidiert und bilden toxische, aliphatische Epoxide. Da bei der üblicherweise angewandten Sterilisationsmethode, die mit einer 3 gew.-%igen Peressigsäure in 30 gew.-%igem Wasserstoffperoxid durchgeführt wird, die entstehende Essigsäure sofort wieder mit dem Wasserstoffperoxid zu Peressigsäure oxidiert wird, wird die Epoxidbildung nicht augenscheinlich.

Die Aufgabe der vorliegenden Erfindung bestand darin, transparente im wesentlichen kompakte, heißdampfsterilisierbare PU-Vergußmassen für medizinisch-technische Artikel zu entwickeln, die die vorgenannten Nachteile nicht aufweisen. Die hierfür in Betracht kommenden PU-Vergußformulierungen sollten bei der Verarbeitung möglichst schaumfrei sein; die erhaltenen Vergußmassen sollten nicht nachhärten und weitgehend wiederstandsfähig sein gegen Percarbonsäuren.

Diese Aufgabe konnte überraschenderweise gelöst werden durch die Verwendung von bei Raumtemperatur flüssigen, mit Urethangruppen speziell modifizierten Diphenylmethan-diisocyanaten oder Diphenylmethan-diisocyanat-Isomerengemischen, vorzugsweise in Kombination mit NCO-Gruppen reaktiven, mindestens difunktionellen Verbindungen, die keine Aminogruppen gebunden enthalten, zur Herstellung der PU-Vergußmassen.

Gegenstand der Erfindung sind somit transparente, heißdampfsterilisierbare, im wesentlichen kompakte Polyurethan-Vergußmassen, die hergestellt werden durch Umsetzung von
A) modifizierten Diphenylmethan-diisocyanaten mit
B) mindestens einer Verbindung mit mindestens zwei reaktiven Wasserstoffatomen, ausgewählt aus der Gruppe der Polyhydroxylverbindungen mit einem Molekulargewicht von 1000 bis 8500 und einer Funktionalität von 2 bis 8 (B1), niedermolekularen zweiwertigen Alkohole, Ester- und/oder Etherbrücken gebunden enthaltenden Glykole (B2) und hydroxylgruppenhaltigen Vernetzer mit einer Hydroxylzahl von 100 bis 1900 und einer Funktionalität von 3 bis 8 (B3), und Mischungen davon
in Gegenwart oder Abwesenheit von
C) Katalysatoren sowie gegebenenfalls
D) Glycerinmonooleat und/oder Glycerindioleat und/oder Blockpolyoxypropylen-polyoxyethylen-glykolen mit einem Gehalt an Ethylenoxideinheiten von 1 bis 80 Gew.-% und einem Molekulargewicht von 1500 bis 8000,
in solchen Mengen, daß das Äquivalenz-Verhältnis von NCO-Gruppen der modifizierten MDI (A) zur Summe der reaktiven Wasserstoffatome der Komponente (B) und gegebenenfalls (D) 1 : 0,9 bis 1 : 1,3 beträgt
und dadurch gekennzeichnet sind, daß man als modifizierte MDI Quasiprepolymere verwendet, die ihrerseits hergestellt werden durch Umsetzung von
A1) 4,4'-MDI oder
A2) 2,4'-MDI oder
A3) einer MDI-Isomerenmischung
mit
A4) mindestens einem Polyoxypropylen-polyol mit einer mittleren Funktionalität von 4 bis 8, vorzugsweise 4 bis 6 und einer Hydroxylzahl von 230 bis 500, vorzugsweise von 250 bis 480, erhalten unter Verwendung von Sucrose oder vorzugsweise Sorbit oder Mischungen aus Sucrose und Sorbit als Starter-moleküle, wobei als Costarter zusätzlich Wasser, Propylenglykol, Glycerin oder Mischungen aus mindestens zwei der genannten Costarter mitverwendet werden können, mit der Maßgabe, daß die Funktionalität der resultierenden Polyoxypropylen-polyole (A4) den Wert 4 nicht unterschreitet, oder
A5) mindestens einem mit Sucrose oder vorzugsweise Sorbit gestarteten Polyoxypropylen-polyoxyethylen-polyol mit einem Gehalt an polymerisierten Ethylenoxideinheiten von 1 bis 80 Gew.-%, vorzugsweise von 10 bis 70 Gew.-%, bezogen auf das Gesamtgewicht, und einer Hydroxylzahl von 150 bis 500, vorzugsweise von 230 bis 300 oder
A6) einer Polyoxyalkylen-polyolmischung mit einer mittleren Funktionalität von mindestens 4 bis 8, vorzugsweise von 4 bis 6, hergestellt aus mindestens einem der vorgenannten Sucrose- oder vorzugsweise Sorbitpolyoxypropylen-(A4)- oder Sucrose- oder vorzugsweise Sorbitpolyoxypropylen-polyoxyethylen-polyole (A5) und einem Polyoxypropylen- und/oder Polyoxyethylen-polyol mit einer Hydroxylzahl von 350 bis 950, vorzugsweise von 380 bis 600, erhalten durch Umsetzung von Glycerin, Trimethylpropan oder einer Mischung aus Glycerin und Trimethylolpropan mit 1,2-Propylenoxid oder Ethylenoxid im Molverhältnis von 1:1 bis 1:8, vorzugsweise von 1:1 bis 1:3, oder Mischungen aus mindestens zwei der Komponenten (A4) bis (A6).
   im Verhältnis von NCO-:OH-Gruppen von 2,5:1 bis 15:1, vorzugsweise von 5:1 bis 10:1.

Gegenstände der Erfindung sind ferner ein Verfahren zur Herstellung der transparenten, heißdampfsterilisierbaren, im wesentlichen kompakten Polyurethan-Vergußmassen gemäß Anspruch 9 und die Verwendung der PU-Vergußmassen zum Einbetten von Hohlfasern aus vorzugsweise Polysulfonen, Polycarbonaten oder Cellulose in Dialysatoren, zur Herstellung von medizinisch-technischen Artikeln sowie zur Bindung von Biokeramikbeschichtungen auf Endoprothesen nach Anspruch 10.

Da im Stand der Technik als geeignete Polyisocyanate zur Herstellung der PU-Vergußmassen, insbesondere zum Einbetten von Hohlfasern in Dialysatoren, neben 1,5-Naphthylen-diisocyanat, Toluylen-diisocyanaten und Phenylen-diisocyanaten auch 4,4'-MDI genannt wird, die Polyisocyanate zweckmäßigerweise in Form von Prepolymeren zur Reaktion gebracht werden und hierbei keine heißdampfsterilisierbaren Polyurethane erhalten werden, war überraschend und nicht vorhersehbar, daß die ausgewählten 4,4'-MDI, 2,4'-MDI oder MDI-Isomerenmischungen, modifiziert mit den speziellen mindestens tetrafunktionellen Polyoxyalkylen-polyolen in bestimmten Mengenverhältnissen, den hieraus hergestellten, ausgehärteten PU-Vergußmassen eine erhöhte Temperaturbeständigkeit und verbesserte Hydrolysebeständigkeit verleihen, so daß die medizinisch-technischen Artikel problemlos heißdampfsterilisiert werden können.

Vorteilhaft ist ferner, daß durch die Verwendung der erfindungsgemäß modifizierten MDI's die maximale Härtungstemperatur der Umsetzung der Komponenten (A) mit (B) in Gegenwart oder vorzugsweise Abwesenheit von (C) deutlich gesenkt werden konnte, so daß bei der Herstellung der PU-Vergußmassen eine Temperatur von 127°C und darüber, gemessen im Mittelpunkt eines konisch sich öffnenden 300 ml Bechers aus Hartpapier (der Firma Uniplast, 7417 Dellingen, BRD) mit einem Bodendurchmesser von ca. 53 mm und einem Öffnungsdurchmesser von ca. 75 mm, in den 100 g Reaktionsmischung eingefüllt werden, nicht erreicht wird, bei der die Hohlfasern aus den vorgenannten Werkstoffen bereits geschädigt werden. Die ausgewählten Komponenten (A) und (B) gewährleisten eine rasche Durchhärtung der PU-Vergußmassen, ohne deren Schnittfähigkeit nach 24 Stunden zu beeinträchtigen. Durch die bevorzugte Verwendung von Verbindungen (B), die außer Hydroxylgruppen, keine weiteren mit NCO-Gruppen reagierenden Gruppen, insbesondere keine primären, sekundären und/oder tertiären Aminogruppen, gebunden enthalten, werden außerdem gegen Percarbonsäuren stabile PU-Vergußmassen erhalten, so daß Formkörper aus derartigen PU-Vergußmassen mit Peressigsäure sterilisiert werden können. Durch den Einsatz von Polyoxypropylenpolyoxyethylen-polyolen mit einem Gehalt an Ethylenoxideinheiten bis zu 80 Gew.-%, vorzugsweise von 10 bis 80 Gew.-%, als Aufbaukomponente (B) können im wesentlichen nichtschäumende PU-Vergußformulierungen hergestellt werden, die vor ihrer Verarbeitung nicht oder nur kurzzeitig entgast werden müssen, was zu einer nicht unerheblichen Reduzierung der Verarbeitungskosten führt.
A) Die erfindungsgemäß verwendbaren modifizierten MDI (A) besitzen bei 23°C zweckmäßigerweise eine Viskosität von 100 bis 8000 m·Pa·s, vorzugsweise von 500 bis 3000 m·Pa·s und einen NCO-Gehalt von 17 bis 29 Gew.%, vorzugsweise von 19 bis 26 Gew.%, bezogen auf das Gesamtgewicht und werden hergestellt nach an sich bekannten Verfahren durch Umsetzung von 4,4'-MDI (A1) oder 2,4'-MDI (A2) oder der MDI-Isomerenmischung (A3) mit mindestens einem Polyoxypropylen-polyol (A4) oder mindestens einem Polyoxypropylen-polyoxyethylen-polyol (A5) oder einer Mischung (A6) aus (A4) oder/und (A5) und mindestens einem mit Glycerin und/oder Trimethylol-propan gestartetem Polyoxypropylen- und/oder Polyoxyethylen-polyol bei einer Temperatur von zweckmäßigerweise 60 bis 100°C, vorzugsweise 70 bis 90°C und in einer Reaktionszeit von 0,5 bis 3 Stunden, vorzugsweise von 1 bis 2 Stunden.
   Als MDI-Isomerenmischungen (A3) finden hierbei zweckmäßigerweise solche Verwendung, die bezogen auf 100 Gew.-Teile, bestehend aus
   A31) 20 bis 90 Gew.-Teilen, vorzugsweise 50 bis 82 Gew.-Teilen 4,4' -MDI,
   A32) 80 bis 8 Gew.-Teilen, vorzugsweise 50 bis 8 Gew.-Teilen 2,4'-MDI und
   A33) 0 bis 5 Gew.-Teilen, vorzugsweise 0 bis 3 Gew.-Teilen 2,2'-MDI.

   Als Polyoxypropylen-polyole (A4) kommen in Betracht: mit Sucrose gestartete Polyoxypropylen-polyole, mit Sorbit gestartete Polyoxypropylen-polyole oder Mischungen der genannten Polyoxypropylen-polyole. Gleichermaßen geeignet sind auch die Polyoxypropylen-polyole, hergestellt unter Verwendung einer Mischung aus Sucrose und Sorbit als Startermoleküle, wobei die Gewichtsverhältnisse von Sucrose zu Sorbit in breiten Grenzen variiert werden können. Vorzugsweise verwendet wird ein mit Sorbit gestartetes Polyoxypropylen-polyol mit einer Hydroxylzahl von 250 bis 380.
   Anstelle von Sucrose oder/und vorzugsweise Sorbit als Startermoleküle können zur Herstellung der Polyoxypropylen-polyole auch Mischungen aus den genannten Startermolekülen und mindestens einem Costarter aus der Gruppe Wasser, Propylenglykol und Glycerin Anwendung finden, mit der Maßgabe, daß die Costarter nur in solchen Mengen eingesetzt werden, daß die Funktionalität der resultierenden Polyoxypropylen-polyole den Wert 4 nicht unterschreitet. Sofern zur Herstellung der Polyoxypropylen-polyole (A4) Costarter mitverwendet werden, werden diese zweckmäßigerweise in solchen Mengen eingesetzt, daß pro Mol Sucrose oder/und Sorbit, maximal 2 Mole, vorzugsweise 0,1 bis 1,8 Mole Costarter in der Startermischung vorliegen.
   Anstelle der vorgenannten Polyoxypropylen-polyole (A4) oder als Mischungen mit diesen, können, wie bereits ausgeführt wurde, auch mit Sucrose oder vorzugsweise mit Sorbit gestartete Polyoxypropylen-polyoxyethylen-polyole (A5) mit einem Gehalt an polymerisierten Ethylenoxideinheiten von 1 bis 80 Gew. -% eingesetzt werden. Geeignet sind ferner Polyoxyalkylen-polyolmischungen (A6) mit einer mittleren Funktionalität von mindestens 4, vorzugsweise 4 bis 8 und insbesondere 4 bis 6 und einer Hydroxylzahl von 230 bis 500, vorzugsweise von 240 bis 480, die zweckmäßigerweise bestehen aus 1 bis 40 Gew.-Teilen, vorzugsweise 3 bis 30 Gew.-Teilen mindestens eines der vorgenannten Sucrose- und/oder Sorbit-polyoxypropylen(A4)- oder -polyoxypropylenpolyoxyethylen-polyole(A5) und 3 bis 30 Gew.-Teilen, vorzugsweise 5 bis 25 Gew.-Teilen mindestens eines der oben beschriebenen Polyoxypropylen-und/oder Polyoxyethylen-polyole auf der Grundlage von Glycerin und/oder Trimethylolpropan als Startermolekülen.
B) Als Verbindungen mit mindestens zwei reaktiven Wasserstoffatomen (B) werden bevorzugt Mischungen verwendet, die bestehen aus
   B1) mindestens einer Polyhydroxylverbindung mit einem Molekulargewicht von 1000 bis 8500 und einer Funktionalität von 2 bis 8,
   B2) mindestens einem niedermolekularen zweiwertigen Alkohol, Ester- und/oder Etherbrücken gebunden enthaltenden Glykol, und
   B3) mindestens einem hydroxylgruppenhaltigen Vernetzer mit einer Hydroxylzahl von 100 bis 1900 und einer Funktionalität von 3 bis 8.

   B1) Als Polyhydroxylverbindungen (B1) mit einem Molekulargewicht von 1000 bis 8500, vorzugsweise von 1500 bis 5600 und insbesondere von 1800 bis 4000 und einer Funktionalität von 2 bis 8, vorzugsweise 2 bis 4 und insbesondere 2 und/oder 3 eignen sich vorzugsweise Polyesterole und insbesondere Polyetherole. In Betracht kommen jedoch auch andere hydroxylgruppenhaltige Polymere mit Ether- oder Estergruppen als Brückenglieder, beispielsweise Polyacetale, wie Polyoxymethylene und vor allem wasserunlösliche Formale, z.B. Polybutandiolformal und Polyhexandiolformal, und Polycarbonate, insbesondere solche aus Diphenylcarbonat und Hexandiol-1,6, hergestellt durch Umesterung. Die genannten Polyhydroxylverbindungen können als Einzelkomponenten oder in Form von Mischungen zur Anwendung kommen.
      Geeignete Polyesterole können beispielsweise aus Dicarbonsäuren mit 2 bis 12, vorzugsweise 4 bis 6 Kohlenstoffatomen und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z.B. in Form einer Bernstein-, Glutar- und Adipinsäuremischung, verwendet werden. Zur Herstellung der Polyesterole kann es gegebenenfalls vorteilhaft sein, anstelle der Dicarbonsäuren die entsprechenden Dicarbonsäurederivate, wie Dicarbonsäuremono- oder -diester mit 1 bis 4 Kohlenstoffatomen im Alkoholrest, Dicarbonsäureanhydride oder Dicarbonsäuredichloride zu verwenden. Beispiele für mehrwertige Alkohole sind Glykole mit 2 bis 10, vorzugsweise 2 bis 6 Kohlenstoffatomen, wie Ethylenglykol, Diethylenglykol, Butandiol-1,4, Pentandiol-1,5, Hexandiol-1,6, Decandiol-1,10, 2,2-Dimethylpropandiol-1,3, Propandiol-1,3 und Dipropylenglykol. Je nach den gewünschten Eigenschaften können die mehrwertigen Alkohole allein oder gegebenenfalls in Mischungen untereinander verwendet werden.
      Geeignet sind ferner Ester der Kohlensäure mit den genannten Diolen, insbesondere solchen mit 4 bis 6 Kohlenstoffatomen, wie Butandiol-1,4 und/oder Hexandiol-1,6, Kondensationsprodukte von ω-Hydroxycarbonsäuren, beispielsweise ω-Hydroxycapronsäure und vorzugsweise Polymerisationsprodukte von Lactonen, beispielsweise gegebenenfalls substituierten ω-Caprolactonen.
      Als Polyesterole vorzugsweise verwendet werden Ethandiol-polyadipate, 1,4-Butandiol-polyadipate, Ethandiol-1,4-butandiol-polyadipate, 1,6-Hexandiol-neopentylglykol-polyadipate, 1,6-Hexandiol-1,4-butandiolpolyadipate und Polycaprolactone.
      Die Polyesterole besitzen Molekulargewichte von 1500 bis 5600, vorzugsweise von 1800 bis 3500.
      Die insbesondere bevorzugt verwendeten Polyetherole können nach bekannten Verfahren, beispielsweise durch anionische Polymerisation mit Alkalihydroxiden, wie Natrium- oder Kaliumhydroxid oder Alkalialkoholaten, wie Natriummethylat, Natrium- oder Kaliumethylat oder Kaliumisopropylat als Katalysatoren und unter Zusatz mindestens eines Startermoleküls das 2 bis 8, vorzugsweise 2 bis 4 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.
      Geeignete Alkylenoxide sind beispielsweise Tetrahydrofuran, 1,3-Propylenoxid, 1,2- bzw. 2,3-Butylenoxid und bevorzugt Ethylenoxid und 1,2-Propylenoxid. Die Alkylenoxide können einzeln, alternierend nacheinander oder als Mischungen verwendet werden. Als Startermoleküle kommen beispielsweise in Betracht: Wasser, organische Dicarbonsäuren, wie Bernsteinsäure, Adipinsäure und/oder Glutarsäure und vorzugsweise zwei- oder dreiwertige, gegebenenfalls Etherbrücken gebunden enthaltende Alkohole, wie z.B. Ethandiol, Propandiol-1,2 und -1,3, Butandiol-1,4, Diethylenglykol, Pentandiol-1,5, Hexandiol-1,6, Dipropylenglykol, 2-Methyl-pentandiol-1,5 und 2-Ethyl-butandiol-1,4, Glycerin, Trimethylolpropan, Pentaerythrit, Sorbit und Saccharose. Die Startermoleküle können einzeln oder als Gemische eingesetzt werden.
      Vorzugsweise verwendet werden Polyetherole aus 1,2-Propylenoxid und Ethylenoxid, in denen mehr als 50 %, vorzugsweise 60 bis 80 % der OH-Gruppen primäre Hydroxylgruppen sind und bei denen zumindest ein Teil des Ethylenoxids als endständiger Block angeordnet ist. Derartige Polyetherole können erhalten werden, indem man z.B. an das Startermolekül zunächst das 1,2-Propylenoxid und daran anschließend das Ethylenoxid polymerisiert oder zunächst das gesamte 1,2-Propylenoxid im Gemisch mit einem Teil des Ethylenoxids copolymerisiert und den Rest des Ethylenoxids anschließend anpolymerisiert oder schrittweise zunächst einen Teil des Ethylenoxids, dann das gesamte 1,2-Propylenoxid und dann den Rest des Ethylenoxids an das Startermolekül anpolymerisiert.
      Insbesondere geeignet sind ferner Polyoxytetramethylen-glykole, vorteilhafterweise solche mit Molekulargewichten von 1000 bis 3000.
      Die geeigneten Polyetherole besitzen Molekulargewichte von 1000 bis 8500, vorzugsweise 1500 bis 5600 und insbesondere 1800 bis 5000. Sie können sowohl einzeln als auch in Form von Mischungen untereinander zur Anwendung kommen.
      Als hydroxylgruppenhaltige Polyacetale kommen z.B. die aus Glykolen, wie Diethylenglykol, Triethylenglykol, 4,4'-Dihydroxyethoxy-diphenyldimethylmethan, Hexandiol und Formaldehyd herstellbaren Verbindungen in Frage. Auch durch Polymerisation cyclischer Acetale lassen sich geeignete Polyacetale herstellen.
      Als Hydroxylgruppen aufweisende Polycarbonate kommen solche der an sich bekannten Art in Betracht, die beispielsweise durch Umsetzung von Diolen, wie Propandiol-1,3, Butandiol-1,4 und/oder Hexandiol-1,6, Diethylenglykol, Triethylenglykol oder Tetraethylenglykol mit Diarylcarbonaten, z.B. Diphenylcarbonat, oder Phosgen hergestellt werden können.
   B2) Als niedermolekulare zweiwertige Alkohole, Ester- oder Ethergruppen als Brückenglieder gebunden enthaltende Glykole kommen z.B. in Betracht: Alkandiole mit 2 bis 10 C-Atomen, vorzugsweise 2 bis 6 C-Atomen, wie z.B. Ethandiol, Propandiol-1,2 oder -1,3, 2,2-Dimethyl-propandiol, Butandiol-1,4, -1,3 oder -2,3, Pentandiol-1,5 oder -2,5, Hexandiol-1,6, 2,2,5-Trimethyl- oder 2,2,5,5-Tetramethyl-hexandiol-1,6; Cycloalkandiole und Alkylcycloalkandiole mit 6 bis 19 C-Atomen, vorzugsweise 6 bis 15 C-Atomen, wie z.B. 1,4-Dihydroxy-cyclohexan, 1-Hydroxymethyl-4-hydroxycyclohexan, 1,4-Bis-(hydroxymethyl)-cyclohexan, 4,4'-Dihydroxy-dicyclohexyl-methan oder -propan-2,2, Esterbrücken gebunden enthaltende Glykole, wie z.B. 3-Hydroxy-2,2-dimethylpropionsäure-2-hydroxyethylester, Terephthalsäure-bis-ethylenglykol oder -butandiol-1,4 und Etherbrücken gebunden enthaltende Glykole mit Molekulargewichten bis 378, wie z.B. Hydroxyalkylenether des Hydrochinons, beispielsweise 1,4-Di-(β-hydroxyethyl)-hydrochinon, Oxyalkylen-glykol mit 4 bis 8 C-Atomen, wie z.B. Diethylen-, Dipropylen- oder Dibutylen-glykol sowie die entsprechenden höhermolekularen Oligomeren davon, wie z.B. Dioxyethylen-, Trioxyethylen-, Dioxypropylen-, Trioxypropylen-, Dioxybutylen-, Trioxybutylen- oder Tetraoxybutylen-glykol sowie ethoxylierte 4,4'-Dihydroxydiphenyl-propane-2,2 mit Molekulargewichten von 316 bis 404. Die Dihydroxyverbindungen aus der Gruppe der Alkan-, Cycloalkan-, Alkylcycloalkan-diole, der entsprechenden Ester- oder Etherbrücken gebunden enthaltenden Glykole und der ethoxylierten 4,4'-Dihydroxydiphenyl-propane-2,2 können einzeln oder als Mischungen eingesetzt werden.
   B3) Als hydroxylgruppenhaltige Vernetzer mit einer Hydroxylzahl von 100 bis 1900 und einer Funktionalität von 3 bis 8 finden vorzugsweise niedermolekulare 3- bis 8-wertige, vorzugsweise 3- bis 4-wertige Alkohole, die entsprechenden Estergruppen als Brückenglied gebunden enthaltende Polyole und die mit niedermolekularen 3- bis 8-wertigen Alkoholen gestartete Polyoxyalkylen-polyole mit Hydroxylzahlen von 100 bis 1900 Verwendung. Beispielhaft genannt seien als 3- und höherwertige Alkohole: Glycerin, Trimethylolpropan, Pentaerythrit, 2,2,6,6-Tetrahydroxymethyl-4-oxaheptandiol-1,7 (Di-pentaerythrit), Tripentaerythrit, 3,3,7,7-Tetrahydroxymethyl-5-oxanon (Di-Trimethylolpropan) und Sorbit.
      Zur Herstellung der Polyoxyalkylen-polyole mit Hydroxylzahlen von 100 bis 1900 finden die vorgenannten 3- bis 8-wertigen Alkohole als Startermoleküle und Ethylenoxid und/oder 1,2-Propylenoxid Verwendung.
      Als hydroxylgruppenhaltige Vernetzer (B3) bewährt haben sich ferner insbesondere und werden daher bevorzugt angewandt, die erfindungsgemäß zur Modifizierung von 4,4'-MDI, 2,4'-MDI oder der MDI-Isomerenmischungen in Betracht kommenden Polyoxypropylen-polyole (A4), Polyoxypropylen-polyoxyethylen-polyole (A5) oder die Polyoxyalkylenmischungen (A6) bestehend aus (A4) und/oder (A5) und mindestens einem mit Glycerin und/oder Trimethylolpropan gestarteten Polyoxypropylen- und/oder Polyoxyethylen-polyol. Besonders bevorzugt sind hierbei Verbindungen mit mindestens zwei reaktiven Wasserstoffatomen (B), die keine primären, sekundären oder tertiären Aminogruppen gebunden enthalten.
      Die Polyhydroxylverbindungen (B1) bis (B3) werden zweckmäßigerweise in solchen Mengen verwendet, daß die Mischung B besteht aus 1,0 Molen (B1), 0,01 bis 48 Molen, vorzugsweise 2 bis 20 Molen (B2) und 0,01 bis 32 Molen, vorzugsweise 1,3 bis 7 Molen (B3).
      Gegebenenfalls kann es vorteilhaft sein, insbesondere wenn PU-Vergußmassen mit hervorragender Oberflächenqualität erforderlich sind, zusätzlich zu den obengenannten Polyhydroxylverbindungen (B1) bis (B3) als weitere Aufbaukomponente (D) Glycerinmonooleat, Glycerindioleat oder Mischungen davon zu verwenden. Werden hingegen als Aufbaukomponente (D) zusätzlich Blockpolyoxypropylen-polyoxyethylen-glykole mit einem Gehalt an Ethylenoxideinheiten von 1 bis 80 Gew.-%, vorzugsweise 10 bis 80 Gew.-% und einem Molekulargewicht von 1500 bis 8000, vorzugsweise von 2000 bis 6000, eingesetzt, derartige Verbindungen sind beispielsweise unter dem Handelsnamen Pluronic®PE der BASF Corporation erhältlich, so werden praktisch nichtschäumende PU-Vergußformulierungen erhalten. Sofern die Glycerinmono- und/oder -diolate und/oder Blockpolyoxypropylen-polyoxyethylen-glykole Anwendung finden, werden diese zweckmäßigerweise in einer Menge von 0,1 bis 5 Gew.%, vorzugsweise von 1 bis 4 Gew.%, bezogen auf das Gesamtgewicht von (A) und (B), eingesetzt.

Die Herstellung der PU-Vergußmassen kann in Gegenwart oder Abwesenheit von Katalysatoren durchgeführt werden. Als geeignete Katalysatoren haben sich Dialkylcarboxylate wie z.B. Dibutylzinndiacetat, Dibutylzinndilaurat sowie dicarboxylierte Dialkylzinnverbindungen, wie sie in der DE-A-3 048 529 beschrieben werden, bewährt. Sofern Katalysatoren eingesetzt werden, werden diese üblicherweise in einer Menge von 0,001 bis 0,2 Gew.-Teilen, vorzugsweise 0,005 bis 0,015 Gew.-Teilen pro 100 Gew.-Teile der Aufbaukomponente (B) verwendet.

Zur Herstellung der PU-Vergußmassen werden die modifizierten MDI (A) und Verbindungen mit mindestens zwei reaktiven Wasserstoffatomen (B) sowie gegebenenfalls die Aufbaukomponente (D) in Gegenwart oder Abwesenheit der Katalysatoren (C) in solchen Mengen zur Umsetzung gebracht, daß das Äquivalenz-Verhältnis von NCO-Gruppen der modifizierten MDI (A) zur Summe der reaktiven Wasserstoffatome der Komponente (B) und gegebenenfalls (D) 1:0,9 bis 1,3, vorzugsweise 1:0,95 bis 1,2 und insbesondere 1:0,98 bis 1,1 beträgt. Hierzu werden die im wesentlichen vollständig entgasten Ausgangskomponenten bei Temperaturen von zweckmäßigerweise 18 bis 70°C, vorzugsweise von 22 bis 60°C intensiv gemischt, die Reaktionsmischung in ein geeignetes Formwerkzeug eingebracht und über einen Zeitraum von 0,3 bis 4 Stunden, vorzugsweise von 1 bis 3 Stunden aushärten gelassen.

Wie bereits dargelegt wurde, finden die transparenten, im wesentlichen kompakten, heißdampfsterilisierbaren PU-Vergußmassen, die vorzugsweise keine Verbindungen (B) mit primären, sekundären und/oder tertiären Aminogruppen gebunden enthalten, insbesondere Verwendung zum Einbetten von Hohlfasern, vorzugsweise von Polysulfon-, Polycarbonat- oder Cellulosehohlfasern in Dialysatoren, wobei die Dialysegeräte, insbesondere die Hülse für das Dialysefilter, zweckmäßigerweise aus einem Polycarbonat auf Basis von Bisphenol A besteht.

Die erfindungsgemäßen PU-Vergußmassen eignen sich ferner zur Herstellung von medizinisch-technischen Artikeln und zur Bindung von Biokeramikbeschichtungen auf Endoprothesen.

Die PU-Vergußmassen sind nicht toxisch, transparent, zeigen keine Wechselwirkung mit den Hohlfasern, besitzen eine starke Haftung zum Polycarbonat und sind ohne Zerstörung der eingebetten Hohlfasern gut zerschneidbar. Wesentlich für die Verwendung der Produkte ist ferner, daß die Maximaltemperatur bei der Aushärtung unter den genannten Bedingungen unter 127°C liegt und die medizinisch-technischen Artikel der Heißdampfsterilisation und anschließenden Trocknung mit einem 80°C heißen Luftstrom unterworfen werden können, ohne daß die ausgehärtete PU-Vergußmasse oder deren Haftung an das Polycarbonatgehäuse geschädigt wird.

### Beispiel 1

### Herstellung des modifizierten MDI

In einem 6 Liter Dreihalskolben wurde eine MDI-Mischung, bestehend aus
3150,94 g 4,4' -MDI und
1050,31 g 2,4'-MDI
auf 80°C erwärmt und hierzu unter Rühren eine Mischung, bestehend aus
399,37 g eines Gemisches mit einer durchschnittlichen Funktionalität von 4,77 und einer Hydroxylzahl von 465, das seinerseits bestand aus 83 Gew.-% eines mit Sorbit und Propylenglykol im Gewichtsverhältnis 92:8 gestarteten Polyoxypropylen-polyols der Hydroxylzahl 490 und 17 Gew.-% eines mit Glycerin gestarteten Polyoxypropylen-polyols mit einer Hydroxylzahl von 400
399,37 g eines mit Glycerin gestarteten Polyoxypropylen-polyols mit einer Hydroxylzahl von 400
über einen Zeitraum von 60 Minuten zutropfen gelassen. Zur Vervollständigung der Umsetzung wurde anschließend noch 60 Minuten bei 80°C gerührt. Das erhaltene modifizierte MDI besaß einen NCO-Gehalt von 22,4 Gew.% und eine Viskosität bei 25°C von 1002 m·Pa·s.

### Beispiel 2

### Herstellung der PU-Vergußmasse

### B-Komponente:

Mischung aus
72,985 Gew.-Teilen eines mit Trimethylolpropan gestarteten Polyoxypropylen (86 Gew.%)-polyoxyethylen (14 Gew.%)-triols mit der Hydroxylzahl 28,
10,0 Gew.-Teilen Butandiol-1,4,
7,0 Gew.-Teilen eines mit Trimethylolpropan gestarteten Polyoxyethylen-triols mit der Hydroxylzahl von 940,
10,0 Gew.-Teilen eines mit Sorbit gestarteten Polyoxypropylen (80 Gew.-%)-polyoxyethylen(20 %)-polyols mit der Hydroxylzahl von 247 und
0,015 Gew.-Teilen Dibutylzinndilaurat.

### A-Komponente:

modifiziertes MDI, hergestellt nach den Angaben des Beispiels 1.

100 Gew.-Teile der Komponente B und 84,51 Gew.-Teile der Komponente A wurden bei 23°C intensiv gemischt, die Reaktionsmischung in ein Formwerkzeug eingegossen und aushärten gelassen.

Die Gelzeit betrug 137 Sekunden und die maximale Reaktionstemperatur 94,3°C, gemessen im Mittelpunkt eines konisch sich öffnenden 300 ml Hartpapierbechers mit einem Bodendurchmesser von 53 mm und einem Öffnungs-durchmesser von 75 mm, in den 100 ml Reaktionsmischung eingefüllt wurden. Die PU-Vergußmasse war transparent und beständig gegen Wasserdampf bei 121°C über einen Zeitraum von mehr als 20 Minuten.

Die Heißdampfsterilisation von Dialysatoren aus Polycarbonat, bestückt mit Polysulfonhohlfasern, hergestellt nach dem Schleudergußverfahren unter Verwendung der PU-Vergußmasse gemäß Beispiel 2, ergab keinerlei Beschädigung, selbst dann nicht, wenn das Dialysefilter nach der Heißdampfsterilisation mit 80°C heißer Luft getrocknet wurde. Die Vergußmasse war gegenüber einer 3 gew.-%igen Peressigsäurelösung bei Raumtemperatur über eine Woche stabil.

### Beispiel 3

### Herstellung des modifizierten MDI

In einem 4-Liter-Dreihalskolben wurde eine MDI-Mischung, bestehend aus
397,02 g 4,4'-MDI und
397,02 g 2,4-MDI
auf 60°C erwärmt und hierzu unter Rühren
204,96 g eines mit Sorbit gestarteten Polyoxypropylen(80 Gew.-%)-polyoxyethylen(20 %)-polyols mit der Hydroxylzahl 242
über einen Zeitraum von 60 Minuten zutropfen gelassen. Anschließend wurde noch 1 Stunde zur Vervollständigung der Reaktion in 30°C nachgerührt. Das erhaltene modifizierte MDI besaß einen NCO-Gehalt von 22,2 Gew.-% und eine Viskosität von 1630 m·Pa·s bei 25°C.

### Beispiel 4

### Herstellung der PU-Vergußmasse

### B-Komponente:

Mischung aus
69,985 Gew.-Teilen eines mit Trimethylolpropan gestarteten Polyoxypropylen (86 Gew.%)-polyoxyethylen (14 Gew.%)-triols mit der Hydroxylzahl 28,
10,0 Gew.-Teilen Butandiol-1,4,
7,0 Gew.-Teilen eines mit Trimethylolpropan gestarteten Polyoxyethylen-triols mit der Hydroxylzahl von 940,
10,0 Gew.-Teilen eines mit Sorbit gestarteten Polyoxypropylen(80 Gew.-%)-polyoxyethylen(20 Gew.-%)-polyols mit der Hydroxylzahl 242,
3,0 Gew.-Teilen eines mit Glycerin gestarteten Polyoxypropylen-triols mit der Hydroxylzahl von 555,
0,015 Gew.-Teilen Dibutylzinndilaurat.

### A-Komponente:

modifiziertes MDI, hergestellt nach den Angaben des Beispiel 3.

Zur Herstellung der PU-Vergußmasse wurden 100 Gew.-Teile der Komponente (B) und 84,51 Gew.-Teile der Komponente (A) bei 23°C intensiv gemischt, die Reaktionsmischung in ein offenes Formwerkzeug eingegossen und aushärten gelassen. Die hierbei gemessene Gelzeit betrug 152 Sekunden und die maximale Reaktionstemperatur 86,5°C, ermittelt analog den Angaben des Beispiels 2.

### Beispiel 5

### Herstellung der PU-Vergußmasse

### B-Komponente:

Mischung aus
72,985 Gew.-Teilen eines mit Trimethylolpropan gestarteten Polyoxypropylen (86 Gew.%)-polyoxyethylen (14 Gew.%)-triols mit der Hydroxylzahl 28,
10,0 Gew.-Teilen Butandiol-1,4,
7,0 Gew.-Teilen eines Polyoxyethylen-triols mit der Hydroxylzahl 940, hergestellt aus 1 Mol Trimethylolpropan als Startermolekül und 1,1 Molen Ethylenoxid,
10,0 Gew.-Teilen eines mit Sorbit gestarteten Polyoxypropylen (80 Gew.-%)-polyoxyethylen(20 %)-polyols mit der Hydroxylzahl von 243 und
0,015 Gew.-Teilen Dibutylzinndilaurat.

### A-Komponente:

modifiziertes MDI, hergestellt nach den Angaben des Beispiels 3.

100 Gew.-Teile der Komponente B und 86,53 Gew.-Teile der Komponente A wurden bei 23°C intensiv gemischt, die Reaktionsmischung in ein Formwerkzeug eingegossen und aushärten gelassen.

Die Gelzeit betrug 157 Sekunden und die maximale Reaktionstemperatur 89°C, gemessen nach den Angaben in Beispiel 2. Die PU-Vergußmasse war transparent und beständig gegen Wasserdampf bei 121°C über einen Zeitraum von mehr als 20 Minuten.

Die Heißdampfsterilisation und anschließende Trocknung mit 80°C heißer Luft von Dialysatoren aus Polycarbonat, bestückt mit Polysulfonhohlfasern, hergestellt nach dem Schleudergußverfahren unter Verwendung der PU-Vergußmasse gemäß Beispiel 5, ergab keinerlei Beschädigung. Die Vergußmasse war gegenüber einer 3 gew.-%igen Peressigsäurelösung bei Raumtemperatur über eine Woche stabil.

## Patentansprüche

1. Transparente, heißdampfsteriliserbare, im wesentlichen kompakte Polyurethan-Vergußmassen, hergestellt durch Umsetzung von
A) modifizierten Diphenylmethan-diisocanaten mit
B) mindestens einer Verbindung mit mindestens zwei reaktiven Wasserstoffatomen, ausgewählt aus der Gruppe der Polyhydroxylverbindungen mit einem Molekulargewicht von 1000 bis 8500 und einer Funktionalität von 2 bis 8 (B1), niedermolekularen zweiwertigen Alkohole, Ester- und/oder Etherbrücken gebunden enthaltenden Glykole (B2) und hydroxylgruppenhaltigen Vernetzer mit einer Hydroxylzahl von 100 bis 1900 und einer Funktionalität von 3 bis 8 (B3),
und Mischungen davon
in Gegenwart oder Abwesenheit von
C) Katalysatoren sowie gegebenenfalls
D) Glycerinmonooleat und/oder Glycerindioleat und/oder Blockpolyoxypropylen-polyoxyethylen-glykolen mit einem Gehalt an Ethylenoxideinheiten von 1 bis 80 Gew.-% und einem Molekulargewicht von 1500 bis 8000,
in solchen Mengen, daß das Äquivalenz-Verhältnis von NCO-Gruppen der modifizierten MDI (A) zur Summe der reaktiven Wasserstoffatome der Komponente (B) und gegebenenfalls (D) 1 : 0,9 bis 1 : 1,3 beträgt
dadurch gekennzeichnet, daß die modifizierten Diphenylmethandiisocyanate (A) hergestellt werden durch Umsetzung von
A1) 4,4'-Diphenylmethan-diisocyanat oder
A2) 2,4'-Diphenylmethan-diisocyanat oder
A3) einer Diphenylmethan-diisocyanat-Isomerenmischung,
mit
A4) mindestens einem Polyoxypropylen-polyol mit einer mittleren Funktionalität von 4 bis 8 und einer Hydroxylzahl von 230 bis 500, erhalten unter Verwendung von Sorbit, Sucrose oder Mischungen aus Sorbit und Sucrose als Startermoleküle, wobei als Costarter zusätzlich Wasser, Propylenglykol, Glycerin oder Mischungen aus mindestens 2 der Costarter mitverwendet werden können, mit der Maßgabe, daß die Funktionalität der resultierenden Polyoxypropylen-polyole (A4) den Wert 4 nicht unterschreitet,
A5) mindestens einem mit Sucrose oder Sorbit gestarteten Polyoxypropylen-polyoxyethylen-polyol mit einem Gehalt an polymerisierten Ethylenoxideinheiten von 1 bis 80 Gew.-%, bezogen auf das Gesamtgewicht, und einer Hydroxylzahl von 150 bis 500 oder
A6) einer Polyoxyalkylen-polyolmischung mit einer mittleren Funktionalität von mindestens 4, hergestellt aus mindestens einem der vorgenannten Sucrose- oder Sorbitpolyoxypropylen-(A4)- oder Sucrose- oder Sorbit-polyoxypropylenpolyoxyethylen-polyole (A5) und einem Polyoxypropylen- und/oder Polyoxyethlyen-polyol mit einer Hydroxylzahl von 350 bis 950, erhalten durch Umsetzung von Glycerin, Trimethylpropan oder einer Mischung aus Glycerin und Trimethylolpropan mit 1,2-Propylenoxid oder Ethylenoxid im Molverhältnis 1 : 1 bis 1 : 8
oder Mischungen aus mindestens zwei der Komponenten (A4) bis (A6), im Verhältnis von NCO- : OH-Gruppen von 2,5 : 1 bis 15 : 1.

2. Transparente, heißdampfsterilisierbare, im wesentlichen kompakte Polyurethan-Vergußmassen nach Anspruch 1, dadurch gekennzeichnet, daß die modifizierten Diphenylmethan-diisocyanate einen NCO-Gehalt von 17 bis 29 Gew.-%, bezogen auf das Gesamtgewicht, besitzen und bei 23°C eine Viskosität von 100 bis 8000 m·Pa·s aufweisen.

3. Transparente, heißdampfsterilisierbare, im wesentlichen kompakte Polyurethan-Vergußmassen nach einem der Ansprüche 1 oder 2, dadurch gekennzeichnet, daß die Diphenylmethan-diisocyanat-Isomerenmischungen, bezogen auf 100 Gew.-Teile, bestehen aus
A31) 20 bis 90 Gew.-Teilen 4,4'-Diphenylmethan-diisocyanat,
A32) 80 bis 8 Gew.-Teilen 2,4'-Diphenylmethan-diisocyanat und
A33) 0 bis 5 Gew.-Teilen 2,2'-Diphenylmethan-diisocyanat.

4. Transparente, heißdampfsterilisierbare, im wesentlichen kompakte Polyurethan-Vergußmassen nach einem der Ansprüche 1 bis 3, dadurch gekennzeichnet, daß als Verbindungen mit mindestens zwei reaktiven Wasserstoffen (B) eine Mischung verwendet wird, die besteht aus
B1) mindestens einer Polyhydroxylverbindung mit einem Molekulargewicht von 1000 bis 8500 und einer Funktionalität von 2 bis 8,
B2) mindestens einem niedermolekularen zweiwertigen Alkohol, Ester- und/oder Etherbrücken gebunden enthaltenden Glykol und
B3) mindestens einem hydroxylgruppenhaltigen Vernetzer mit einer Hydroxylzahl von 100 bis 1900 und einer Funktionalität von 3 bis 8.

5. Transparente, heißdampfsterilisierbare, im wesentlichen kompakte Polyurethan-Vergußmassen nach einem der Ansprüche 1 bis 4, dadurch gekennzeichnet, daß die Verbindungen mit mindestens zwei reaktiven Wasserstoffatomen (B) keine primären, sekundären oder tertiären Aminogruppen gebunden enthalten.

6. Transparente, heißdampfsterilisierbare, im wesentlichen kompakte Polyurethan-Vergußmassen, nach einem Ansprüche 1 bis 3, dadurch gekennzeichnet, daß als Verbindungen mit mindestens zwei reaktiven Wasserstoffatomen (B) eine Mischung verwendet wird, die besteht aus
B1) mindestens einem Polyetherol mit einem Molekulargewicht von 1000 bis 8500 und einer Funktionalität von 2 bis 8,
B2) mindestens einer niedermolekularen Dihydroxyverbindung, ausgewählt aus der Gruppe der Alkan-, Cycloalkan-, Alkylcycloalkandiole, der entsprechenden Ester- oder Ethergruppen als Brückenglied gebunden enthaltenden Glykole und ethoxylierten 4,4'-Dihydroxydiphenyl-propane-2,2 mit Molekulargewichten von 316 bis 404 und
B3) mindestens einem Vernetzer aus der Gruppe der niedermolekularen 3- bis 8-wertigen Alkohole, der entsprechenden Estergruppen als Brückenglied gebunden enthaltenden Polyole und der mit niedermolekularen 3- bis 8-wertigen Alkoholen gestarteten Polyoxyalkylen-polyole mit Hydroxylzahlen von 100 bis 1900
und wobei die Verbindungen (B) keine Gruppen mit primären, sekundären oder tertiären Stickstoffatomen gebunden enthalten.

7. Transparente, heißdampfsterilisierbare, im wesentlichen kompakte Polyurethan-Vergußmassen nach einem der Ansprüche 1 bis 6, dadurch gekennzeichnet, daß (B) aus einer Mischung besteht aus
1,0 Molen (B1),
0,01 bis 48 Molen (B2) und
0,01 bis 32 Molen (B3).

8. Transparente, heißdampfsterilisierbare, im wesentlichen kompakte Polyurethan-Vergußmassen nach einem der Ansprüche 1 bis 7, dadurch gekennzeichnet, daß zu ihrer Herstellung als Aufbaukomponente (D) zusätzlich verwendet werden 0,1 bis 5 Gew.-%, bezogen auf das Gesamtgewicht von (A) und (B), Glycerinmono- und/oder -dioleat und/oder Blockpolyoxypropylen-polyoxyethylen-glykole mit einem Gehalt an Ethylenoxideinheiten von 1 bis 80 Gew.-% und einem Molekulargewicht von 1500 bis 8000.

9. Verfahren zur Herstellung von transparenten, heißdampfsterilisierbaren, im wesentlichen kompakten Polyurethan-Vergußmassen durch Umsetzung von
A) modifizierten Diphenylmethan-diisocyanaten mit
B) mindestens einer Verbindung mit mindestens zwei reaktiven Wasserstoffatomen, ausgewählt aus der Gruppe der Polyhydroxylverbindungen mit einem Molekulargewicht von 1000 bis 8500 und einer Funktionalität von 2 bis 8 (B1), niedermolekularen zweiwertigen Alkohole, Ester- und/oder Etherbrücken gebunden enthaltenden Glykole (B2) und hydroxylgruppenhaltigen Vernetzer mit einer Hydroxylzahl von 100 bis 1900 und einer Funktionalität von 3 bis 8 (B3),
und Mischungen davon
in Gegenwart oder Abwesenheit von
C) Katalysatoren sowie gegebenenfalls
D) Glycerinmonooleat und/oder Glycerindioleat und/oder Blockpolyoxypropylen-polyoxyethylen-glykolen mit einem Gehalt an Ethylenoxideinheiten von 1 bis 80 Gew.-% und einem Molekulargewicht von 1500 bis 8000,
in solchen Mengen, daß das Äquivalenz-Verhältnis von NCO-Gruppen der modifizierten MDI (A) zur Summe der reaktiven Wasserstoffatome der Komponente (B) und gegebenenfalls (D) 1 : 0,9 bis 1 : 1,3 beträgt
dadurch gekennzeichnet, daß die modifizierten Diphenylmethandiisocyanate (A) bei 23°C eine Viskosität von 100 bis 8000 m·Pa·s aufweisen, einen NCO-Gehalt von 17 bis 29 Gew.-%, bezogen auf das Gesamtgewicht, besitzen und hergestellt werden durch Umsetzung von
A1) 4,4'-Diphenylmethan-diisocyanat oder
A2) 2,4'-Diphenylmethan-diisocyanat oder
A3) einer Diphenylmethan-diisocyanat-Isomerenmischung, bestehend aus, bezogen auf 100 Gew.-Teile,
A31)20 bis 90 Gew.-Teilen 4,4'-Diphenylmethan-diisocyanat,
A32)80 bis 8 Gew.-Teilen 2,4'-Diphenylmethan-diisocyanat und
A33)0 bis 15 Gew.-Teilen 2,2'-Diphenylmethan-diisocyanat
mit
A4) mindestens einem Polyoxypropylen-polyol mit einer mittleren Funktionalität von 4 bis 8 und einer Hydroxylzahl von 230 bis 500, erhalten unter Verwendung von Sorbit, Sucrose oder Mischungen aus Sorbit und Sucrose als Startermoleküle, wobei als Costarter zusätzlich Wasser, Propylenglykol, Glycerin oder Mischungen aus mindestens 2 der Costarter mitverwendet werden können, mit der Maßgabe, daß die Funktionalität der resultierenden Polyoxypropylen-polyole (A4) den Wert 4 nicht unterschreitet,
A5) mindestens einem mit Sucrose oder Sorbit gestarteten Polyoxypropylen-polyoxyethylen-polyol mit einem Gehalt an polymerisierten Ethylenoxideinheiten von 1 bis 80 Gew.-%, bezogen auf das Gesamtgewicht, und einer Hydroxylzahl von 150 bis 500 oder
A6) einer Polyoxyalkylen-polyolmischung mit einer mittleren Funktionalität von mindestens 4, hergestellt aus mindestens einem der vorgenannten Sucrose- oder Sorbitpolyoxypropylen-(A4)- oder Sucrose- oder Sorbit-polyoxypropylenpolyoxyethylen-polyole (A5) und einem Polyoxypropylen- und/oder Polyoxyethlyen-polyol mit einer Hydroxylzahl von 350 bis 950, erhalten durch Umsetzung von Glycerin, Trimethylpropan oder einer Mischung aus Glycerin und Trimethylolpropan mit 1,2-Propylenoxid oder Ethylenoxid im Molverhältnis 1 : 1 bis 1 : 8
oder Mischungen aus mindestens zwei der Komponenten (A4) bis (A6), im Verhältnis von NCO- : OH-Gruppen von 2,5 : 1 bis 15 : 1.

10. Verwendung der transparenten, heißdampfsterilisierbaren, im wesentlichen kompakten Polyurethan-Vergußmassen nach einem der Ansprüche 1 bis 8 zum Einbetten von Hohlfasern aus vorzugsweise Polysulfonen, Polycarbonaten oder Cellulose in Dialysegeräte, zur Herstellung von medizinisch-technischen Artikeln sowie zur Bindung von Biokeramikbeschichtungen auf Endoprothesen.

## Claims

1. A transparent, steam-sterilizable, essentially compact polyurethane encapsulating composition, prepared by reacting
A) modified diphenyl methane diisocyanates with
B) at least one compound containing at least two reactive hydrogen atoms, selected from the group consisting of polyhydroxyl compounds having a molecular weight of from 1000 to 8500 and a functionality of from 2 to 8 (B1), low-molecular-weight, dihydric alcohols or glycols containing bonded ester and/or ether bridges (B2), and hydroxyl-containing crosslinking agents having a hydroxyl number of from 100 to 1900 and a functionality of from 3 to 8 (B3), and mixtures thereof,
in the presence or absence of
C) catalysts, and, if desired,
D) glycerol monooleate and/or glycerol dioleate and/or block polyoxypropylene-polyoxyethylene glycols containing from 1 to 80% by weight of ethylene oxide units and having a molecular weight of from 1500 to 8000,
in such amounts that the ratio between the number of equivalents of NCO groups in the modified MDI (A) and the total number of reactive hydrogen atoms in component (B) and, if used, (D) is from 1:0.9 to 1:1.3,
wherein the modified diphenylmethane diisocyanates (A) are prepared by reacting
A1) 4,4'-diphenylmethane diisocyanate or
A2) 2,4'-diphenylmethane diisocyanate or
A3) a diphenylmethane diisocyanate isomer mixture
with
A4) at least one polyoxypropylene-polyol having a mean functionality of from 4 to 8 and a hydroxyl number of from 230 to 500, obtained using sorbitol, sucrose or a mixture of sorbitol and sucrose as initiator molecules, it additionally being possible to use, as coinitiator, water, propylene glycol, glycerol or a mixture of at least two of the coinitiators, with the proviso that the functionality of the resultant polyoxypropylene-polyols (A4) is not less than 4,
A5) at least one sucrose- or sorbitol-initiated polyoxypropylene-polyoxyethylene-polyol containing from 1 to 80% by weight of polymerized ethylene-oxide units, based on the total weight, and having a hydroxyl number of from 150 to 500, or
A6) a polyoxyalkylene-polyol mixture having a mean functionality of at least 4, prepared from at least one of the abovementioned sucrose or sorbitol polyoxypropylene-polyols (A4) or sucrose or sorbitol polyoxypropylene-polyoxyethylene-polyols (A5) and a polyoxypropylene-polyol and/or polyoxyethylene-polyol having a hydroxyl number of from 350 to 950, obtained by reacting glycerol, trimethylolpropane or a mixture of glycerol and trimethylolpropane with 1,2-propylene oxide or ethylene oxide in a molar ratio of from 1:1 to 1:8,
or mixtures of at least two of components (A4) to (A6), in an NCO:OH group ratio of from 2.5:1 to 15:1.

2. A transparent, steam-sterilizable, essentially compact polyurethane encapsulating composition as claimed in claim 1, wherein the modified diphenylmethane diisocyanates contain from 17 to 29% by weight of NCO, based on the total weight, and have a viscosity of from 100 to 8000 m·Pa·s at 23°C.

3. A transparent, steam-sterilizable, essentially compact polyurethane encapsulating composition as claimed in claim 1 or 2, wherein the diphenylmethane diisocyanate isomer mixtures comprise, based on 100 parts by weight,
A31) from 20 to 90 parts by weight of 4,4'-diphenylmethane diisocyanate,
A32) from 80 to 8 parts by weight of 2,4'-diphenylmethane diisocyanate
A33) from 0 to 5 parts by weight of 2,2'-diphenylmethane diisocyanate.

4. A transparent, steam-sterilizable, essentially compact polyurethane encapsulating composition as claimed in any of claims 1 to 3, wherein the compounds (B) containing at least two reactive hydrogen atoms are a mixture comprising
B1) at least one polyhydroxyl compound having a molecular weight of from 1000 to 8500 and a functionality of from 2 to 8,
B2) at least one low-molecular-weight, dihydric alcohol or glycol containing bonded ester and/or ether bridges, and
B3) at least one hydroxyl-containing crosslinking agent having a hydroxyl number of from 100 to 1900 and a functionaility of from 3 to 8.

5. A transparent, steam-sterilizable, essentially compact polyurethane encapsulating composition as claimed in any of claims 1 to 4, wherein the compounds (B) containing at least two reactive hydrogen atoms contain no bonded primary, secondary or tertiary amino groups.

6. A transparent, steam-sterilizable, essentially compact polyurethane encapsulating composition as claimed in any of claims 1 to 3, wherein the compounds (B) containing at least two reactive hydrogen atoms are a mixture comprising
B1) at least one polyether-ol having a molecular weight of from 1000 to 8500 and a functionality of from 2 to 8,
B2) at least one low-molecular-weight dihydroxyl compound selected from the group consisting of alkanediols, cycloalkanediols, alkylcycloalkanediols, the corresponding glycols containing bonded ester or ether groups as bridging members, and ethoxylated 4,4'-dihydroxydiphenyl-2,2-propanes having molecular weights of from 316 to 404, and
B3) at least one crosslinking agent from the group consisting of low-molecular-weight, trihydric to octahydric alcohols, the corresponding polyols containing bonded ester groups as bridging members, and polyoxylalkylene-polyols initiated by means of low-molecular-weight, trihydric to octahydric alcohols, having hydroxyl numbers of from 100 to 1900,
where the compounds (B) contain no bonded groups containing primary, secondary or tertiary nitrogen atoms.

7. A transparent, steam-sterilizable, essentially compact polyurethane encapsulating composition as claimed in any of claims 1 to 6, wherein (B) comprises a mixture of
1.0 mol of (B1),
from 0.01 to 48 mol of (B2) and
from 0.01 to 32 mol of (B3).

8. A transparent, steam-sterilizable, essentially compact polyurethane encapsulating composition as claimed in any of claims 1 to 7, wherein formative component (D) additionally used for its preparation is from 0.1 to 5% by weight, based on the total weight of (A) and (B), of glycerol monooleate and/or dioleate and/or block polyoxypropylene-polyoxyethylene glycols containing from 1 to 80% by weight of ethylene oxide units and having a molecular weight of from 1500 to 8000.

9. A process for the preparation of transparent, steam-sterilizable, essentially compact polyurethane encapsulating compositions by reacting
A) modified diphenylmethane diisocyanates with
B) at least one compound containing at least two reactive hydrogen atoms, selected from the group consisting of polyhydroxyl compounds having a molecular weight of from 1000 to 8500 and a functionality of from 2 to 8 (B1), low-molecular-weight, dihydric alcohols or glycols containing bonded ester and/or ether bridges (B2), and hydroxyl-containing crosslinking agents having a hydroxyl number of from 100 to 1900 and a functionality of from 3 to 8 (B3), and mixtures thereof,
in the presence or absence of
C) catalysts, and, if desired,
D) glycerol monooleate and/or glycerol dioleate and/or block polyoxypropylene-polyoxyethylene glycols containing from 1 to 80% by weight of ethylene oxide units and having a molecular weight of from 1500 to 8000,
in such amounts that the ratio between the number of equivalents of NCO groups in the modified MDI (A) and the total number of reactive hydrogen atoms in component (B) and, if used, (D) is from 1:0.9 to 1:1.3, wherein the modified diphenylmethane diisocyanates (A) have a viscosity of from 100 to 8000 m·Pa·s at 23°C, contain from 17 to 29% by weight of NCO, based on the total weight, and are prepared by reacting
A1) 4,4'-diphenylmethane diisocyanate or
A2) 2,4'-diphenylmethane diisocyanate or
A3) a diphenylmethane diisocyanate isomer mixture, comprising, based on 100 parts by weight,
A31) from 20 to 90 parts by weight of 4,4'-diphenylmethane diisocyanate,
A32) from 80 to 8 parts by weight of 2,4'-diphenylmethane diisocyanate and
A33) from 0 to 15 parts by weight of 2,2'-diphenylmethane diisocyanate,
with
A4) at least one polyoxypropylene-polyol having a mean functionality of from 4 to 8 and a hydroxyl number of from 230 to 500, obtained using sorbitol, sucrose or a mixture of sorbitol and sucrose as initiator molecules, it additionally being possible to use, as coinitiator, water propylene glycol, glycerol or a mixture of at least two of the coinitiators, with the proviso that the functionality of the resultant polyoxypropylene-polyols (A4) is not less than 4,
A5) at least one sucrose- or sorbitol-initiated polyoxypropylene-polyoxyethylene-polyol containing from 1 to 80% by weight of polymerized ethylene-oxide units, based on the total weight, and having a hydroxyl number of from 150 to 500, or
A6) a polyoxyalkylene-polyol mixture having a mean functionality of at least 4, prepared from at least one of the abovementioned sucrose or sorbitol polyoxypropylene-polyols (A4) or sucrose or sorbitol polyoxypropylene-polyoxyethylene-polyols (A5) and a polyoxypropylene-polyol and/or polyoxyethylene-polyol having a hydroxyl number of from 350 to 950, obtained by reacting glycerol, trimethylolpropane or a mixture of glycerol and trimethylolpropane with 1,2-propylene oxide or ethylene oxide in a molar ratio of from 1:1 to 1:8,
or mixtures of at least two of components (A4) to (A6), in an NCO:OH group ratio of from 2.5:1 to 15:1.

10. The use of a transparent, steam-sterilizable, essentially compact polyurethane encapsulating composition as claimed in any of claims 1 to 8 for embedding hollow fibers, preferably made from polysulfones, polycarbonates or cellulose, in dialysis equipment, for the production of medical technology articles and for bonding bioceramic coatings to endoprostheses.

## Revendications

1. Matières à mouler par coulée, en polyuréthannes transparentes, stérilisables par la vapeur à chaud, essentiellement compactes, préparées par réaction de
A) des diphénylméthane-diisocyanates modifiés avec
B) au moins un composé contenant au moins deux atomes d'hydrogène réactifs, choisi dans le groupe des composés polyhydroxylés de poids moléculaire 1000 à 8500 et de fonctionnalité 2 à 8 (B1), des alcools divalents à bas poids moléculaire, des glycols contenant à l'état chimiquement combiné des ponts ester et/ou éther (B2) et des agents réticulants contenant des groupes hydroxy, ayant un indice d'hydroxyle de 100 à 1900 et une fonctionnalité de 3 à 8 (B3), et leurs mélanges
en présence ou en l'absence de
C) des catalyseurs et le cas échéant
D) du mono-oléate de glycérol et/ou du dioléate de glycérol et/ou des polyoxypropylène-polyoxyéthylène-glycols séquencés d'une teneur en motifs d'oxyde d'éthylène de 1 à 80 % en poids et un poids moléculaire de 1500 à 8000,
en quantités telles que le rapport entre les équivalents des groupes NCO du MDI modifié (A) et la somme des atomes d'hydrogène réactifs du composant (B) et le cas échéant du composant (D) soit de 1 : 0,9 à 1 : 1,3,
caractérisé en ce que les diphénylméthane-diisocyanates modifiés (A) ont été préparés par réaction de
A1) le 4,4'-diphénylméthane-diisocyanate ou bien
A2) le 2,4'-diphénylméthane-diisocyanate ou bien
A3) un mélange de diphénylméthane-diisocyanates isomères,
avec
A4) au moins un polyoxypropylène-polyol de fonctionnalité moyenne 4 à 8 et d'indice d'hydroxyle 230 à 500, obtenu avec utilisation de sorbitol, de saccharose ou de mélanges de sorbitol et de saccharose en tant que molécules de départ, avec le cas échéant utilisation conjointe de molécules auxiliaires de départ consistant en eau, propylène-glycol, glycérol ou mélanges d'au moins deux de ces molécules auxiliaires de départ, sous réserve que la fonctionnalité des polyoxypropylène-polyols (A4) ainsi obtenus ne soit pas inférieure à 4,
A5) au moins un polyoxypropylène-polyoxyéthylène-polyol condensé sur une mole de départ de saccharose ou de sorbitol, d'une teneur en motifs d'oxyde d'éthylène polymérisés de 1 à 80 % en poids par rapport au poids total, et d'un indice d'hydroxyle de 150 à 500, ou bien
A6) un mélange de polyoxyalkylène-polyols de fonctionnalité moyenne d'au moins 4, préparé à partir d'au moins un des saccharose- ou sorbitol-polyoxypropylènes- (A4)- ou saccharose- ou sorbitol-polyoxypropylène-polyoxyéthylène-polyols (A5) mentionnés ci-dessus, et d'un polyoxypropylène- et/ou polyoxyéthylène-polyol d'indice d'hydroxyle 350 à 950, obtenu par réaction du glycérol, du triméthylolpropane ou d'un mélange de glycérol et de triméthylolpropane avec l'oxyde de 1,2-propylène ou l'oxyde d'éthylène dans un rapport molaire de 1 : 1 à 1 : 8,
ou des mélanges d'au moins deux des composants (A4) à (A6), en un rapport de 2,5 : 1 à 15 :1 entre les groupes NCO et les groupes OH.

2. Matières à mouler par coulée, en polyuréthannes transparentes, stérisables par la vapeur à chaud, essentiellement compactes, selon la revendication 1, caractérisées en ce que les diphénylméthane-diisocyanates modifiés ont une teneur en NCO de 17 à 29 % en poids par rapport au poids total, et une viscosité de 100 à 8000 m.Pa.s à 23°C.

3. Matières à mouler par coulée, en polyuréthannes transparentes, stérisables à la vapeur à chaud, essentiellements compactes, selon l'une des revendications 1 ou 2, caractérisées en ce que les mélanges de diphénylméthane-diisocyanates isomères consistent, pour 100 parties en poids, en
A31) 20 à 90 parties en poids de 4,4'-diphénylméthanediisocyanate,
A32) 80 à 8 parties en poids de 2,4'-diphénylméthanediisocyanate et
A33) 0 à 5 parties en poids de 2,2'-diphénylméthanediisocyanate.

4. Matières à mouler par coulée, en polyuréthannes transparentes, stérisables à la vapeur à chaud, essentiellement compactes, selon l'une des revendications 1 à 3, caractérisées en ce que l'on utilise en tant que composés contenant au moins deux atomes d'hydrogène réactifs (B) un mélange consistant en
B1) au moins un polyéther-ol de poids moléculaire 1000 à 8500 et de fonctionnalité 2 à 8,
B2) au moins un alcool divalent à bas poids moléculaire ou glycol contenant à l'état chimiquement combiné des ponts ester et/ou éther et
B3) au moins un agent réticulant contenant des groupes hydroxy, d'indice d'hydroxyle 100 à 1900 et de fonctionnalité 3 à 8.

5. Matières à mouler par coulée, en polyuréthannes transparentes, stérilisables à la vapeur à chaud, essentiellement compactes, selon l'une des revendications 1 à 4, caractérisées en ce que les composés contenant au moins deux atomes d'hydrogène réactifs (B) ne contiennent pas de groupes amino primaires, secondaires ou tertiaires à l'état chimiquement combiné.

6. Matières à mouler par coulée, en polyuréthannes transparentes, stérilisables à la vapeur à chaud, essentiellement compactes, selon l'une des revendications 1 à 3, caractériséés en ce que l'on utilise en tant que composés contenant au moins deux atomes d'hydrogène réactifs (B) un mélange consistant en
B1) au moins un polyéther-ol de poids moléculaire 1000 à 8500 et de fonctionnalité 2 à 8,
B2) au moins un composé dihydroxylé à bas poids moléculaire choisi dans le groupe formé par les alcane-, les cycloalcane-, les alkylcycloalcane-diols, les glycols correspondants contenant à l'état chimiquement combiné des ponts ester ou éther et des 4,4'-dihydroxydiphényl-propanes-2,2 éthoxylés, de poids moléculaire 316 à 404 et
B3) au moins un agent réticulant du groupe des alcools à bas poids moléculaire tri- à octo-valents, des polyols correspondants contenant à l'état chimiquement combiné des ponts ester et des polyoxyalkylène-polyols condensés sur une mole de départ d'alcool tri- à octo-valent, à bas poids moléculaire, et d'indice d'hydroxyle 100 à 1900, les composés (B) ne contenant pas de groupes à atomes d'azote primaires, secondaires ou tertiaires à l'état chimiquement combiné.

7. Matières à mouler par coulée, en polyuréthannes transparentes, stérilisable à la vapeur à chaud, essentiellement compactes, selon une des revendications 1 à 6, caractérisées en ce que (B) consiste en un mélange de
1,0 mol de (B1),
0,01 à 48 mol de (B2) et
0,01 à 32 mol de (B3).

8. Matières à mouler par coulée, en polyuréthannes transparentes, stérilisables à la vapeur à chaud, essentiellement compactes, selon une des revendications 1 à 7, caractérisées en ce que, pour leur préparation, on utilise en outre en tant que composant de synthèse (D) de 0,1 à 5 % en poids, par rapport au poids total de (A) et (B), de mono- et/ou de di-oléate de glycérol et/ou de polyoxypropylène-polyoxyéthylène-glycols séquencés d'une teneur en motifs d'oxyde d'éthylène de 1 à 80 % en poids et un poids moléculaire de 1500 à 8000.

9. Procédé de préparation d'une matière à mouler par coulée, en polyuréthanne transparente, stérilisable à la vapeur à chaud, essentiellement compacte, par réaction de
A) des diphénylméthane-diisocyanates modifiés avec
B) au moins un composé contenant au moins deux atomes d'hydrogène réactifs, choisi dans le groupe des composés polyhydroxylés de poids moléculaire 1000 à 8500 et de fonctionnalité 2 à 8 (B1), des alcools divalents à bas poids moléculaire, des glycols contenant à l'état chimiquement combiné des ponts ester et/ou éther (B2) et des agents réticulants contenant des groupes hydroxy, d'indice d'hydroxyle 100 à 1900 et de fonctionnalité 3 à 8 (B3),
et leurs mélanges,
en présence ou en l'absence de
C) des catalyseurs et le cas échéant
D) du mono-oléate de glycérol et/ou du dioléate de glycérol et/ou des polyoxypropylène-polyoxyéthylène-glycols séquencés d'une teneur en motifs d'oxyde d'éthylène de 1 à 80 % en poids et un poids moléculaire de 1500 à 8000,
en quantités telles que le rapport entre les équivalents de groupes NCO du MDI modifié (A) avec la somme des atomes d'hydrogène réactifs du composant (B) et le cas échéant (D), soit de 1 : 0,9 à 1 : 1,3,
caractérisé en ce que les diphénylméthane-diisocyanates modifiés (A) ont, à 23°C, une viscosité de 100 à 800 m.Pa.s, une teneur en NCO de 17 à 29 % en poids, par rapport au poids total, et ont été préparés par réaction de
A1) le 4,4'-diphénylméthane-diisocyanate ou bien
A2) le 2,4'-diphénylméthane-diisocyanate ou bien
A3) un mélange de diphénylméthane-diisocyanates isomères consistant, pour 100 parties en poids, en
A31) 20 à 90 parties en poids de 4,4'-diphénylméthanediisocyanate,
A32) 80 à 8 parties en poids de 2,4'-diphénylméthanediisocyanate, et
A33) 0 à 15 parties en poids de 2,2'-diphénylméthanediisocyanate
avec
A4) au moins un polyoxypropylène-polyol de fonctionnalité moyenne 4 à 8 et d'indice d'hydroxyle 230 à 500, obtenu avec utilisation du sorbitol, du saccharose ou d'un mélange de sorbitol et de saccharose en tant que molécule de départ, avec le cas échéant des molécules auxiliaires de départ consistant en eau, propylène-glycol, glycérol ou un mélange d'au moins deux de ces molécules auxiliaires de départ, sous réserve que la fonctionnalité des polyoxypropylène-polyols (A4) obtenus ne soit pas inférieure à 4,
A5) au moins un polyoxypropylène-polyoxyéthylène-polyol condensé sur une mole de départ de saccharose ou de sorbitol, d'une teneur en motifs d'oxyde d'éthylène polymérisés de 1 à 80 % en poids, par rapport au poids total, et un indice d'hydroxyle de 150 à 500, ou bien
A6) un mélange de polyoxyalkylène-polyols de fonctionnalité moyenne au moins 4, préparé à partir d'au moins un des saccharose- ou sorbitol-polyoxypropylène- (A4)- ou saccharose- ou sorbitol-polyoxypropylène-polyoxyéthylène-polyols (A5) mentionnés ci-dessus et d'un polyoxypropylène- et/ou polyoxyéthylène-polyol d'indice d'hydroxyle 350 à 950, obtenu par réaction du glycérol, du triméthylolpropane ou d'un mélange de glycérol et de triméthylolpropane avec l'oxyde de 1,2-propylène ou l'oxyde d'éthylène dans un rapport molaire de 1 : 1 à 1 : 8,
ou des mélanges d'au moins deux des composants (A4) à (A6), en un rapport de 2,5 : 1 à 15 : 1 entre les groupes NCO et les groupes OH.

10. Utilisation des matières à mouler par coulée, en polyuréthannes transparentes, stérilisables à la vapeur à chaud, essentiellement compactes, selon une des revendications 1 à 8 pour l'enrobage de fibres creuses consistant de préférence en polysulfones, polycarbonates ou cellulose dans des appareils de dialyse, pour la fabrication d'articles techniques médicinaux ou pour la fixation de revêtements de biocéramique sur des endoprothèses.
